(19) Europäisches Patentamt
European Patent Office
Office européen des brevets

(11) Numéro de publication: **0 516 547 B1**

(12) **FASCICULE DE BREVET EUROPEEN**

(45) Date de publication de fascicule du brevet: **19.04.95**

(51) Int. Cl.[6]: **A61K 7/00**, A61K 7/48, B01F 17/00

(21) Numéro de dépôt: **92401464.0**

(22) Date de dépôt: **27.05.92**

# (54) Emulsion stable du huile-dans-l'eau à base d'huile de silicone et son utilisaton en cosmétique et en dermatologie.

(30) Priorité: **27.05.91 FR 9106345**

(43) Date de publication de la demande:
**02.12.92 Bulletin 92/49**

(45) Mention de la délivrance du brevet:
**19.04.95 Bulletin 95/16**

(84) Etats contractants désignés:
**BE DE ES FR GB IT**

(56) Documents cités:
**EP-A- 0 076 146**
**EP-A- 0 152 953**
**EP-A- 0 154 837**
**EP-A- 0 200 916**
**EP-A- 0 291 683**
**GB-A- 2 206 048**

(73) Titulaire: **L'OREAL**
**14, Rue Royale**
**F-75008 Paris (FR)**

(72) Inventeur: **Gregoire, Nathalie**
**40, avenue Franklin Roosevelt**
**F-92330 Sceaux (FR)**
Inventeur: **Boelle, Anne**
**107, rue de Paris**
**F-92190 Meudon (FR)**

(74) Mandataire: **Stalla-Bourdillon, Bernard et al**
**CABINET NONY & CIE**
**29, rue Cambacérès**
**F-75008 Paris (FR)**

**EP 0 516 547 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet une émulsion stable du type huile-dans-eau à base d'huile de silicone et son utilisation dans le domaine cosmétique ou dermatologique, notamment dans des crèmes, des laits ou encore des produits de maquillage tels que mascaras, fonds de teint ou eye-liners.

Les huiles de silicone constituent des ingrédients particulièrement recherchés en cosmétique car elles permettent de conférer d'excellentes propriétés lubrifiantes et waterproof.

Par ailleurs, par rapport aux huiles classiques, les huiles de silicone forment un film protecteur sur la peau la protégeant d'une déshydratation sans que l'on observe un effet de gras.

Jusqu'à présent les études qui ont été réalisées n'ont pas permis d'incorporer de façon satisfaisante des proportions appréciables d'huiles de silicone dans des émulsions du type huile-dans-eau. En effet, l'incorporation d'huiles de silicone est limitée à environ 15 % maximum et les huiles de silicone sont des huiles de silicone cycliques volatiles et des polydiméthylsiloxanes (silicones linéaires) de faible viscosité (< 100 centistokes).

D'autres études ont été entreprises dans le but d'obtenir des émulsions du type huile-dans-eau à base d'huiles de silicone en utilisant divers tensioactifs en particulier des tensioactifs nonioniques et anioniques.

On peut à cet égard citer les émulsions huile-dans-eau à base d'huile de silicone décrites dans le brevet US 4 917 891, le brevet français n° 2 485 923, le brevet européen EP 76 146 et le brevet US 4 788 001.

Il s'est toutefois avéré selon ces brevets que l'emploi de tensioactifs classiques ne permettait pas toujours d'obtenir une bonne émulsification des huiles de silicone dans l'eau.

Après d'importantes recherches on vient maintenant de constater de façon surprenante et inattendue, qu'il était tout à fait possible d'obtenir des émulsions stables du type huile-dans-eau à base d'huile de silicone.

Selon l'invention, l'obtention de telles émulsions du type huile-dans-eau est rendue possible grâce à l'emploi d'un agent émulsionnant particulier du type polyorganosiloxane polyoxyalkyléné présentant des caractéristiques bien déterminées.

Ce type d'agent émulsionnant est en effet dispersible dans l'eau et insoluble dans la phase huile à base d'huile de silicone.

La présente invention a donc pour objet une émulsion cosmétique ou dermatologique stable du type huile-dans-eau, à base d'huile de silicone, constituée d'une phase aqueuse, d'une phase huileuse et d'un agent émulsionnant, la phase huileuse étant essentiellement constituée d'au moins une huile de silicone et l'agent émulsionnant étant un polyorganosiloxane polyoxyéthyléné répondant à la formule suivante :

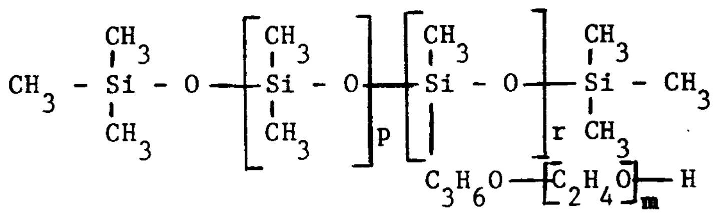

(I)

dans laquelle :

p est un nombre compris entre 15 et 35 de préférence entre 20 et 30,

r est un nombre compris entre 2 et 6

m est un nombre compris entre 5 et 15 de préférence entre 8 et 12,

et p, r et m étant tels que le rapport du poids moléculaire de la chaîne latérale polyoxyéthylénée/channe polysiloxane est d'environ 50/50.

Selon l'invention le polyorganosiloxane polyoxyéthyléné de formule (I) doit avoir de préférence un HLB (Hydrophilic Lipophilic Balance) compris entre 9 et 12 et un poids moléculaire compris entre 2 000 et 7 000 et de préférence entre 3 500 et 4 500.

Selon une forme de réalisation préférée de l'invention le polyorganosiloxane polyoxyéthyléné est un polydiméthylsiloxane polyoxyéthyléné ayant un poids moléculaire de 4 000 et un HLB de 10, avec un rapport du poids moléculaire de la chaîne latérale polyoxyéthylénée/chaîne polysiloxane de 50/50.

Diverses études comparatives ont été effectuées et ont permis de mettre en évidence l'importance des caractéristiques de l'agent émulsionnant de formule (I) en vue d'obtenir des émulsions stables.

Selon l'invention la proportion de l'agent émulsionnant de formule (I) est généralement comprise entre 1 et 10 % en poids et de préférence entre 4 et 6 % par rapport au poids total de l'émulsion.

La phase aqueuse de l'émulsion représente de 50 à 75 % en poids et de préférence de 60 à 70 % en poids par rapport au poids total de l'émulsion.

La phase huileuse de l'émulsion représente de 20 à 50 % en poids et de préférence de 30 à 40 % en poids par rapport au poids total de l'émulsion.

Dans cette phase huileuse la proportion d'huile de silicone est supérieure à 50 % et de préférence comprise entre 70 et 100 % en poids.

L'émulsion selon l'invention peut également contenir de 0,05 à 20 % en poids d'un agent gélifiant de la phase aqueuse.

Parmi les agents gélifiants on peut notamment citer les dérivés de cellulose, les polysaccharides les polymères acryliques tels qu'un Carbomer ou un polyacrylate de glycéryle.

Selon l'invention l'agent gélifiant permet d'améliorer encore la stabilité de l'émulsion.

Comme mentionné ci-dessus la phase huileuse de l'émulsion est essentiellement constituée d'une huile de silicone qui est une huile de silicone volatile cyclique ou un polydiméthylsiloxane de faible viscosité. Parmi les huiles de silicone volatiles ont peut notamment citer le cyclopentadiméthylsiloxane et le cyclotétradiméthylsiloxane.

Lorsque la phase huileuse n'est pas totalement constituée d'une huile de silicone, celle-ci peut contenir des huiles végétales, animales, minérales, ou synthétiques.

Parmi les huiles végétales, on peut citer l'huile de jojoba, l'huile d'olive, l'huile d'amande douce, l'huile d'avocat, l'huile de coco, l'huile de germe de blé, l'huile de maïs, l'huile de palme, l'huile de sésame, l'huile de soja, l'huile d'argan, l'huile d'onagre, l'huile de bourrache et les huiles essentielles.

Parmi les huiles animales on peut notamment citer l'huile de poisson.

Parmi les huiles minérales on peut citer l'huile de vaseline et d'isohexadécane.

Parmi les huiles synthétiques on peut mentionner les palmitates d'éthyle et d'isopropyle, le palmitate d'éthyl-2 hexyle, les myristates d'alkyle tels que le myristate d'isopropyle, de butyle, de cétyle, le stéarate d'hexyle, les triglycérides des acides octanoïque et décanoïque, le ricinoléate de cétyle et l'octanoate de stéaryle.

La phase huileuse peut par ailleurs contenir des colorants, des filtres solaires, des antioxydants, des conservateurs et des principes actifs lipophiles.

La phase aqueuse de l'émulsion peut contenir en plus du gélifiant, des dérivés hydrosolubles en particulier des colorants, des conservateurs, des actifs hydrosolubles et des hydratants tels que la glycérine.

L'émulsion selon l'invention peut également contenir des parfums, des huiles essentielles, des pigments, des charges, des vitamines, et diverses autres substances actives au sens de la cosmétique ou de la dermatologie.

Les émulsions selon l'invention sont préparées par dispersion du tensioactif de formule (I) dans l'eau et addition lente de la phase huileuse dans cette dispersion aqueuse en agitant fortement. Quand l'émulsion contient un gélifiant, celui-ci est ajouté selon sa nature, soit avant l'addition de la phase huileuse soit après formation de l'émulsion.

Les émulsions selon l'invention peuvent être utilisées en cosmétique et en dermatologie pour des crèmes pour le visage, pour le corps ou pour le cuir chevelu et la chevelure, pour des laits démaquillants ou des laits corporels ou capillaires. Ces émulsions peuvent être également utilisées dans des produits de maquillage après addition de pigments tels que dans des mascaras, fonds de teint et eye-liners.

On va maintenant donner à titre d'illustration et sans aucun caractère limitatif plusieurs exemples d'émulsions huile-dans-eau selon l'invention.

EXEMPLE 1 : Crème gélifiée de soin pour le visage sous forme d'une émulsion huile-dans-eau.

| | |
|---|---|
| . Polydiméthylsiloxane polyoxyéthyléné de formule (I) (PM 4000 et HLB 10) | 5 % |
| . "Pemulen TR2" vendu par la Société GOODRICH | 0,1 % |
| . Triéthanolamine | 0,1 % |
| . Cyclopentadiméthylsiloxane | 25 % |
| . Huile de jojoba | 10 % |
| . Eau...q.s.p | 100 % |

Après étalement de cette crème sur la peau, elle pénètre bien ; elle est douce et apporte une grande fraîcheur.

EXEMPLE 2 : Lait corporel sous forme d'une émulsion huile-dans-eau.

| | |
|---|---|
| . Polydiméthysiloxane polyoxyéthyléné de formule (I) (PM 4000, HLB 10) | 4 % |
| . Polysaccharide de glucose-mannose-acide glucuronique. (Keltrol de la Société KELCO) | 1 % |
| Cyclopentadiméthylsiloxane | 30 % |
| . Eau...q.s.p | 100 % |

Ce lait corporel confère une sensation très fraîche lors de l'application.

Ce lait corporel confère une sensation très fraîche lors de l'application.

EXEMPLE 3 : Fond de teint sous forme d'une émulsion huile dans l'eau

| | |
|---|---|
| . Polydiméthylsiloxane polyoxyéthyléné de Formule (I) (PM = 4000 et HLB 10) | 4.80 % |
| . Gel de polyacrylate de glycéryle dans l'eau (50/50) | 4.50 % |
| . Gomme de xanthane | 0.20 % |
| . Cyclopentadiméthylsiloxane | 22.60 % |
| . Huile de jojoba | 9.00 % |
| . Oxyde de fer jaune | 0.95 % |

- . Oxydes de fer brun, jaune ........................... 0.35 %
- . Oxyde de fer noir ................................... 0.15 %
- . Oxyde de titane ..................................... 3.25 %
- . Méthylparaben ....................................... 0.20 %
- . Eau ................................................. 100 %

Ce fond de teint s'applique facilement, donne un effet naturel et est frais à l'application.

## Revendications

1. Emulsion cosmétique stable du type huile-dans-eau constituée d'une phase aqueuse, d'une phase huileuse et d'un agent émulsionnant, caractérisée par le fait que la phase huileuse est essentiellement constituée d'au moins une huile de silicone et que l'agent émulsionnant est un polyorganosiloxane polyoxyéthyléné répondant à la formule suivante :

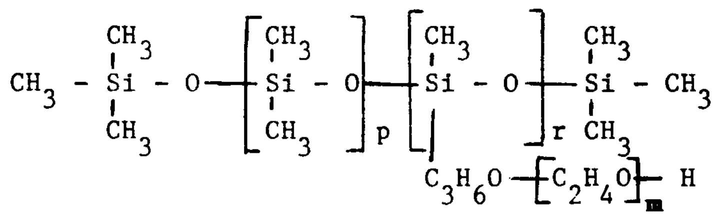

(I)

dans laquelle :
p est un nombre compris entre 15 et 35 de préférence entre 20 et 30,
r est un nombre compris entre 2 et 6
m est un nombre compris entre 5 et 15 de préférence entre 8 et 12,
et p, r et m étant tels que le rapport du poids moléculaire de la chaîne latérale polyoxyéthylénée/chaîne polysiloxane est d'environ 50/50,
le HLB du dit agent émulsionnant étant compris entre 9 et 12.

2. Emulsion selon la revendication 1, caractérisée par le fait que l'huile de silicone est choisie parmi les huiles de silicone cycliques volatiles et les polydiméthylsiloxanes de faible viscosité.

3. Emulsion selon la revendication 1, caractérisée par le fait que le polyorganosiloxane polyoxyéthyléné de formule (I) a un poids moléculaire compris entre 2.000 et 7.000 et de préférence entre 3.500 et 4.500.

4. Emulsion selon la revendication 1 ou 3, caractérisée par le fait que le polyorganopolysiloxane polyoxyéthyléné a un poids moléculaire de 4.000, un HLB de 10 et un rapport du poids moléculaire de la chaîne latérale polyoxyéthylénée/chaine polysiloxane de 50/50.

5. Emulsion selon l'une quelconque des revendications précédentes, caractérisée par le fait que la proportion de l'agent émulsionnant est comprise entre 1 et 10 % en poids et de préférence entre 4 et 6 % par rapport au poids total de l'émulsion.

6. Emulsion selon la revendication 1, caractérisée par le fait que la phase aqueuse de l'émulsion représente de 50 à 75 % en poids et de préférence de 60 à 70 % par rapport au poids total de l'émulsion.

7. Emulsion selon la revendication 1, caractérisée par le fait que la phase huileuse de l'émulsion représente de 20 à 50 % en poids et de préférence de 30 à 40 % par rapport au poids total de l'émulsion.

**8.** Emulsion selon la revendication 1, 2 ou 7, caractérisée par le fait que la phase huileuse contient l'huile de silicone en une proportion supérieure à 50 % et de préférence comprise entre 70 et 100 % en poids.

**9.** Emulsion selon l'une quelconque des revendications précédentes, caractérisée par le fait qu'elle contient en outre de 0,05 à 20 % en poids d'un agent gélifiant.

**10.** Emulsion selon l'une quelconque des revendications précédentes, caractérisée par le fait que la phase huileuse contient en outre des colorants, des filtres solaires, des antioxydants, des conservateurs et des principes actifs lipophiles.

**11.** Emulsion selon l'une quelconque des revendications 1 à 9, caractérisée par le fait que la phase aqueuse contient en outre des colorants, des conservateurs, des actifs hydrosolubles et des agents hydratants.

## Claims

**1.** Stable cosmetic emulsion of the oil-in-water type consisting of an aqueous phase, an oily phase and an emulsifying agent, characterized in that the oily phase consists essentially of at least one silicone oil and in that the emulsifying agent is a polyoxyethylenated polyorganosiloxane corresponding to the following formula:

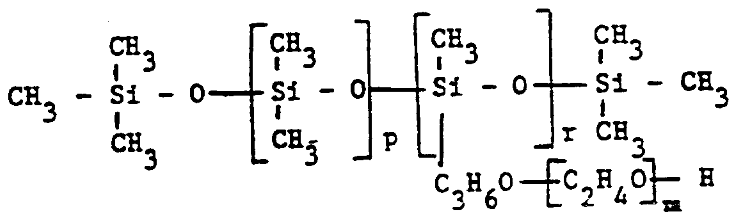

(I)

in which:

p is a number between 15 and 35, preferably between 20 and 30,

r is a number between 2 and 6

m is a number between 5 and 15, preferably between 8 and 12,

and p, r and m being such that the molecular weight ratio of the polyoxyethylenated side chain/polysiloxane chain is approximately 50/50,

the HLB of the said emulsifying agent being between 9 and 12.

**2.** Emulsion according to Claim 1, characterized in that the silicone oil is chosen from volatile cyclic silicone oils and polydimethylsiloxanes of low viscosity.

**3.** Emulsion according to Claim 1, characterized in that the polyoxyethylenated polyorganosiloxane of formula (I) has a molecular weight of between 2000 and 7000 and preferably of between 3500 and 4500.

**4.** Emulsion according to Claim 1 or 3, characterized in that the polyoxyethylenated polyorganopolysiloxane has a molecular weight of 4000, an HLB of 10 and a molecular weight ratio of the polyoxyethylenated side chain/polysiloxane chain of 50/50.

**5.** Emulsion according to any one of the preceding claims, characterized in that the proportion of the emulsifying agent is between 1 and 10% by weight and preferably between 4 and 6% relative to the total weight of the emulsion.

**6.** Emulsion according to Claim 1, characterized in that the aqueous phase of the emulsion represents from 50 to 75% by weight and preferably from 60 to 70% relative to the total weight of the emulsion.

**7.** Emulsion according to Claim 1, characterized in that the oily phase of the emulsion represents from 20 to 50% by weight and preferably from 30 to 40% relative to the total weight of the emulsion.

**8.** Emulsion according to Claim 1, 2 or 7, characterized in that the oily phase contains the silicone oil in a proportion of greater than 50% and preferably between 70 and 100% by weight.

**9.** Emulsion according to any one of the preceding claims, characterized in that it additionally contains from 0.05 to 20% by weight of a gelling agent.

**10.** Emulsion according to any one of the preceding claims, characterized in that the oily phase additionally contains dyes, sunscreen agents, antioxidants, preserving agents and lipophilic active principles.

**11.** Emulsion according to any one of Claims 1 to 9, characterized in that the aqueous phase additionally contains dyes, preserving agents, water-soluble active agents and moisturizing agents.

**Patentansprüche**

**1.** Stabile, kosmetische Emulsion vom Typ Öl-in-Wasser, welche aus einer wäßrigen Phase, einer Ölphase und einem Emulgator besteht, dadurch gekennzeichnet, daß die Ölphase im wesentlichen aus mindestens einem Silikonöl gebildet wird und daß der Emulgator ein Polyorganosiloxan-Polyoxyethylen entsprechend der folgenden allgemeinen Formel darstellt:

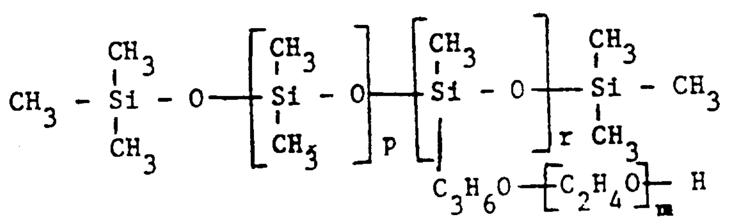

(I)

in der
p eine ganze Zahl von 15 bis 35, vorzugsweise von 20 bis 30,
r eine ganze Zahl von 2 bis 6 sowie
m eine ganze Zahl von 5 bis 15, vorzugsweise von 8 bis 12 bedeuten und
p, r und m so gewählt sind, daß das Molekulargewichtsverhältnis der Polyoxyethylenseitenkette zur Polysiloxankette ungefähr 50/50 beträgt, wobei der HLB-Wert des Emulgators von 9 bis 12 beträgt.

**2.** Emulsion gemäß Anspruch 1, dadurch gekennzeichnet, daß das Silikonöl unter den flüchtigen Ölen cyclischer Silikone und den Polydimethylsiloxanen niedriger Viskosität ausgewählt ist.

**3.** Emulsion gemäß Anspruch 1, dadurch gekennzeichnet, daß das Polyorganosiloxan-Polyoxyethylen der Formel (I) ein Molekulargewicht zwischen 2000 und 7000 und vorzugsweise zwischen 3500 und 4500 aufweist.

**4.** Emulsion gemäß Anspruch 1 oder 3, dadurch gekennzeichnet, daß das Polyorganosiloxan-Polyoxyethylen ein Molekulargewicht von 4000, einen HLB-Wert von 10 und Molekulargewichtsverhältnis der Polyoxyethylenseitenkette zur Polysiloxankette von 50/50 aufweist.

**5.** Emulsion gemäß einem der vorstehenden Ansprüche, dadurch gekennzeichnet, daß der Anteil an Emulgator zwischen 1 und 10 Gew.-% und vorzugsweise zwischen 4 und 6 Gew.-%, bezogen auf das Gesamtgewicht der Emulsion, beträgt.

**6.** Emulsion gemäß Anspruch 1, dadurch gekennzeichnet, daß die wäßrige Phase 50 bis 75 Gew.-% und vorzugsweise 60 bis 70 %, bezogen auf das Gesamtgewicht der Emulsion, ausmacht.

**7.** Emulsion gemäß Anspruch 1, dadurch gekennzeichnet, daß die Ölphase 20 bis 50 Gew.-% und vorzugsweise 30 bis 40 %, bezogen auf das Gesamtgewicht der Emulsion, ausmacht.

**8.** Emulsion gemäß einem der Ansprüche 1, 2 oder 7, dadurch gekennzeichnet, daß die Ölphase einen Anteil an Silikonöl von mehr als 50 % und vorzugsweise von 70 bis 100 Gew.-% enthält.

**9.** Emulsion gemäß einen der vorstehenden Ansprüche, dadurch gekennzeichnet, daß sie zusätzlich von 0,05 bis 20 Gew.-% Gelierungsmittel enthält.

**10.** Emulsion gemäß einen der vorstehenden Ansprüche, dadurch gekennzeichnet, daß die Ölphase außerdem Farbstoffe, Sonnenschutzmittel, Antioxidanzien, Konservierungsmittel und lipophile Wirkstoffe enthält.

**11.** Emulsion gemäß einem der Ansprüche 1 bis 9, dadurch gekennzeichnet, daß die wäßrige Phase zusätzlich Farbstoffe, Konservierungsmittel, wasserlösliche Wirkstoffe und Feuchthaltemittel enthält.